# EUROPEAN PATENT APPLICATION

(11) **EP 2 072 523 A1**
(43) Date of publication of application: **24.06.2009**
(21) Application number: 07024713.5
(22) Date of filing: 20.12.2007
(51) Int. Cl.: C07H 15/08, A01N 25/30

(54) **Alkylene oxide adducts of oligosaccharides**

(71) Applicant: Cognis IP Management GmbH, 40589 Düsseldorf (DE)
(72) Inventor: Mainx, Hans-Georg, 42799 Leichlingen (DE); Hofer, Peter, 40589 Düsseldorf (DE)
(74) Representative: Reinhardt, Jürgen

(57) **Abstract**

Suggested are alkylene oxide adducts of oligosaccharides, obtainable in that an aqueous solution of at least one water-soluble oligosaccharide is subjected to alkoxylation within a temperature range of 90 to 160 °C.

## Description

### Field of the invention

The present invention is related to the area of agriculture and concerns new non-ionic sugar surfactants, a process for making them and their use as additives, in particular as adjuvants or formulation aids in plant and crop protection.

### Background of the invention

Alkoxylation products of sugar alcohols, for example sorbitol, are known for long. These products are very well known ad adjuvants and formulation aids for pesticides in crop and plant protection. Nevertheless, it is desirous to replace sugar alcohols by oligosaccharides, such as saccharose, since these raw materials are much cheaper and easier available in the market. However, so far alkoxylation of oligosaccharides is difficult for the following reasons:
(i) standard alkoxylation processes need temperature significantly above 110°C; under these conditions however, oligosaccharides start to decompose ("caramelisation"):
(ii) at lower temperatures selectivity is poor due to the formation of high amounts of unwanted polyglycolethers;
(iii) alkoxylation of solids requires significant higher afford than alkoxylation of liquids.

Therefore, the object of the present invention has been to provide alkoxylation products of oligosaccharides showing a decreased amount of polyglycolethers (PGE), in particular PGE-amounts of less than 5, preferably less than 2 and most preferably less than 1 % b.w. and a process for obtaining them which avoids the disadvantages of the state of the art.

### Detailed description of the invention

The present invention refers to alkylene oxide adducts of oligosaccharides, obtainable in that an aqueous solution of at least one water-soluble oligosaccharide is subjected to alkoxylation within a temperature range of 90 to 160 °C.

Surprisingly it has been observed that conducting alkoxylation of oligosaccharides in the presence of water, in particular using saturated or nearly saturated aqueous solutions of said carbohydrates allows raising the temperature up to 160 °C without any or very little decomposition or discolouring of the starting material. At the same time the formation of unwanted polyglycolethers is reduced to amounts of less than 5, in most cases less than 1 % b.w. Since the starting material is liquid alkoxylation can take place in standard equipments.

As second object of the present invention covers a process for making alkylene oxide adducts of oligosaccharides, which is characterised in that an aqueous solution of at least one water-soluble oligosaccharide is subjected to alkoxylation within a temperature range of 90 to 160 °C.

### Water-soluble oligosaccharides

Basically, the selection of oligosaccharides is little critical as long as they show at room temperature a sufficient solubility or at least dispensability in water. An oligosaccharide is considered to be water soluble in case solubility is at least 100 g/L. Therefore the choice fully depends on the properties of the final alkoxylation product in the proposed application. Suitable candidates are disaccharides, trisaccharides and oligosaccharides having at least 4 and on average not more than 20 sugar units. The saccharides may represent oligoglucosides or oligofructoside or even mixtures of both structures. Preferred examples are selected from the group comprising saccharose, maltose, and maltotriose. For economic reasons suitable candidates for alkoxylation can also be obtained by chemical or enzymatic degradation of polysaccharides like for example celluloses, starches or waste material from sugar industry. Also certain natural or synthetic gums or their degradation products, like for example xanthan gum are useful.

Although it is also possible to work with diluted solutions of said oligosaccharides it is for economic reasons more useful to prepare saturated aqueous solutions; it counts to the surprising effects associated with the present invention that even solutions of about 300 g oligosaccharide in 100 ml water can subjected to alkoxylation at temperatures of about 120 °C without any or even very little decomposition or caramelisation.

### Alkylene oxides

Alkoxylation can be conducted by adding ethylene oxide (EO), propylene oxide (PO) or their mixture to the hydroxyl groups of the oligosaccharides. As a matter of fact alkoxylation is driven by statistics that means adding for example 10 moles ethylene oxide to saccharose will lead to a complex mixture of mono, di and tri-substituted adducts, each of them showing different degrees of ethoxylation. The alkoxylation may take place blockwise or randomised, that means it is possible to add first an amount of ethylene oxide and then propylene oxide to the hydroxyl groups in order to produce blocks of EO and PO units, or to use mixtures of both alkylene oxide in order to an irregular sequence of EO and PO groups. On average about 1 to about 50, preferably about 5 to about 25 and more preferably about 10 to 20 mol alkylene oxide are added to each Mol of oligosaccharide. Preferred products are adducts of about 10 to 12 mol ethylene oxide to saccharose, maltose or maltotriose.

### Alkoxylation process

Basically alkoxylation is conducted in known manner which for example also standard for the production of fatty alcohol polyglycolethers. The aqueous solutions containing the oligosaccharides along with the catalyst are placed into a pressure reactor. Suitable catalysts are alkaline bases, like sodium hydroxide, potassium hydroxide or sodium methylate, which are used in the form of concentrated or diluted aqueous or alcoholic solutions. Typically the catalysts are used in amounts of about 1 to about 5 % b.w. calculated on the sum of starting materials. It has been found advantageous to conduct alkoxylation within a temperature interval of 90 to 160, preferably 100 to 140 °C and a pressure in between 1 and 5 bar. Once the addition of the alkylene oxide is completed it is advantageous to stir the product for another 30 minutes while maintaining the temperature. Subsequently, the reactor is cooled down, vacuum is broken and the catalyst is neutralised by adding an organic acid, such as for example lactic acid.

### Industrial application

Another object of the present invention refers to the use of the new alkylene oxide adducts of oligosaccharides as additives in agriculture, in particular as adjuvants, tank-mix adjuvants or formulation aids for making plant and crop protection compositions.

Typically, the new alkylene oxide adducts are used together with biocides in rations by weight - calculated on the active matter (= adjuvant + biocide) - of about 1:99 to about 50:50, preferably about 10:80 to about 40:60. These compositions are also object of the present invention.

### Biocides

A biocide is a chemical substance capable of killing different forms of living organisms used in fields such as medicine, agriculture, forestry, and mosquito control. Usually, biocides are divided into two sub-groups:
- pesticides, which includes fungicides, herbicides, insecticides, algicides, moluscicides, miticides and rodenticides, and
- antimicrobials, which includes germicides, antibiotics, antibacterials, antivirals, antifungals, antiprotozoals and antiparasites.

Biocides can also be added to other materials (typically liquids) to protect the material from biological infestation and growth. For example, certain types of quaternary ammonium compounds (quats) can be added to pool water or industrial water systems to act as an algicide, protecting the water from infestation and growth of algae. Suitable biocides which can be combined with the alkylene oxide adducts according to the present invention are described in the following. The present invention includes the observation that due to the low amount of unwanted by-products such as polyalkyleneglycol ethers, the new alkylene oxide adducts show an improved synergistic behavior when combined with said biocides, especially species like glyphosate, glyfusinate, paraquat and their mixtures.

### Pesticides

The U.S Environmental Protection Agency (EPA) defines a pesticide as "any substance or mixture of substances intended for preventing, destroying, repelling, or mitigating any pest".^{[1]} A pesticide may be a chemical substance or biological agent (such as a virus or bacteria) used against pests including insects, plant pathogens, weeds, mollusks, birds, mammals, fish, nematodes (roundworms) and microbes that compete with humans for food, destroy property, spread disease or are a nuisance. In the following examples, pesticides suitable for the agrochemical compositions according to the present invention are given:

***Fungicides.*** A fungicide is one of three main methods of pest control - the chemical control of fungi in this case. Fungicides are chemical compounds used to prevent the spread of fungi in gardens and crops. Fungicides are also used to fight fungal infections. Fungicides can either be contact or systemic. A contact fungicide kills fungi when sprayed on its surface. A systemic fungicide has to be absorbed by the fungus before the fungus dies. Examples for suitable fungicides, according to the present invention, encompass the following species: (3-ethoxypropyl)mercury bromide, 2-methoxyethylmercury chloride, 2-phenylphenol, 8-hydroxyquinoline sulfate, 8-phenylmercurioxyquinoline, acibenzolar, acylamino acid fungicides, acypetacs, aldimorph, aliphatic nitrogen fungicides, allyl alcohol, amide fungicides, ampropylfos, anilazine, anilide fungicides, antibiotic fungicides, aromatic fungicides, aureofungin, azaconazole, azithiram, azoxystrobin, barium polysulfide, benalaxy,l benalaxyl-M, benodanil, benomyl, benquinox, bentaluron, benthiavalicarb, benzalkonium chloride, benzamacril, benzamide fungicides, benzamorf, benzanilide fungicides, benzimidazole fungicides, benzimidazole precursor fungicides, benzimidazolylcarbamate fungicides, benzohydroxamic acid, benzothiazole fungicides, bethoxazin, binapacryl, biphenyl, bitertanol, bithionol, blasticidin-S, Bordeaux mixture, boscalid, bridged diphenyl fungicides, bromuconazole, bupirimate, Burgundy mixture, buthiobate, butylamine, calcium polysulfide, captafol, captan, carbamate fungicides, carbamorph, carbanilate fungicides, carbendazim, carboxin, carpropamid, carvone, Cheshunt mixture, chinomethionat, chlobenthiazone, chloraniformethan, chloranil, chlorfenazole, chlorodinitronaphthalene, chloroneb, chloropicrin, chlorothalonil, chlorquinox, chlozolinate, ciclopirox, climbazole, clotrimazole, conazole fungicides, conazole fungicides (imidazoles), conazole fungicides (triazoles), copper(II) acetate, copper(II) carbonate, basic, copper fungicides, copper hydroxide, copper naphthenate, copper oleate, copper oxychloride, copper(II) sulfate, copper sulfate, basic, copper zinc chromate, cresol, cufraneb, cuprobam, cuprous oxide, cyazofamid, cyclafuramid, cyclic dithiocarbamate fungicides, cycloheximide, cyflufenamid, cymoxanil, cypendazole, cyproconazole, cyprodinil, dazomet, DBCP, debacarb, decafentin, dehydroacetic acid, dicarboximide fungicides, dichlofluanid, dichlone, dichlorophen, dichlorophenyl, dicarboximide fungicides, dichlozoline, diclobutrazol, diclocymet, diclomezine, dicloran, diethofencarb, diethyl pyrocarbonate, difenoconazole, diflumetorim, dimethirimol, dimethomorph, dimoxystrobin, diniconazole, dinitrophenol fungicides, dinobuton, dinocap, dinocton, dinopenton, dinosulfon, dinoterbon, diphenylamine, dipyrithione, disulfiram, ditalimfos, dithianon, dithiocarbamate fungicides, DNOC, dodemorph, dodicin, dodine, donatodine, drazoxolon, edifenphos, epoxiconazole, etaconazole,etem, ethaboxam, ethirimol, ethoxyquin, ethylmercury 2,3-dihydroxypropyl mercaptide, ethylmercury acetate, ethylmercury bromide, ethylmercury chloride, ethylmercury phosphate, etridiazole, famoxadone, fenamidone, fenaminosulf, fenapanil, fenarimol, fenbuconazole, fenfuram, fenhexamid, fenitropan, fenoxanil, fenpiclonil, fenpropidin, fenpropimorph, fentin, ferbam, ferimzone, fluazinam, fludioxonil, flumetover, flumorph, fluopicolide, fluoroimide, fluotrimazole, fluoxastrobin, fluquinconazole, flusilazole, flusulfamide, flutolanil, flutriafol, folpet, formaldehyde, fosetyl, fuberidazole, furalaxyl, furametpyr, furamide fungicides, furanilide fungicides, furcarbanil, furconazole, furconazole-cis, furfural, furmecyclox, furophanate, glyodin, griseofulvin, guazatine, halacrinate, hexachlorobenzene, hexachlorobutadiene, hexachlorophene, hexaconazole, hexylthiofos, hydrargaphen, hymexazol, imazalil, imibenconazole, imidazole fungicides, iminoctadine, inorganic fungicides, inorganic mercury fungicides, iodomethane, ipconazole, iprobenfos, iprodione, iprovalicarb, isoprothiolane, isovaledione, kasugamycin, kresoxim-methyl, lime sulphur, mancopper, mancozeb, maneb, mebenil, mecarbinzid, mepanipyrim, mepronil, mercuric chloride, mercuric oxide, mercurous chloride, mercury fungicides, metalaxyl, metalaxyl-M, metam, metazoxolon, metconazole, methasulfocarb, methfuroxam, methyl bromide, methyl isothiocyanate, methylmercury benzoate, methylmercury dicyandiamide, methylmercury pentachlorophenoxide, metiram, metominostrobin, metrafenone, metsulfovax, milneb, morpholine fungicides, myclobutanil, myclozolin, N-(ethylmercury)-p-toluenesulphonanilide, nabam, natamycin, nitrostyrene, nitrothal-isopropyl, nuarimol, OCH, octhilinone, ofurace, organomercury fungicides, organophosphorus fungicides, organotin fungicides, orysastrobin, oxadixyl, oxathiin fungicides, oxazole fungicides, oxine copper, oxpoconazole, oxycarboxin, pefurazoate, penconazole, pencycuron, pentachlorophenol, penthiopyrad, phenylmercuriurea, phenylmercury acetate, phenylmercury chloride, phenylmercury derivative of pyrocatechol, phenylmercury nitrate, phenylmercury salicylate, phenylsulfamide fungicides, phosdiphen, phthalide, phthalimide fungicides, picoxystrobin, piperalin, polycarbamate, polymeric dithiocarbamate fungicides, polyoxins, polyoxorim, polysulfide fungicides, potassium azide, potassium polysulfide, potassium thiocyanate, probenazole, prochloraz, procymidone, propamocarb, propiconazole, propineb, proquinazid, prothiocarb, prothioconazole, pyracarbolid, pyraclostrobin, pyrazole fungicides, pyrazophos, pyridine fungicides, pyridinitril, pyrifenox, pyrimethanil, pyrimidine fungicides, pyroquilon, pyroxychlor, pyroxyfur, pyrrole fungicides, quinacetol, quinazamid, quinconazole, quinoline fungicides, quinone fungicides, quinoxaline fungicides, quinoxyfen, quintozene, rabenzazole, salicylanilide, silthiofam, simeconazole, sodium azide, sodium orthophenylphenoxide, sodium pentachlorophenoxide, sodium polysulfide, spiroxamine, streptomycin, strobilurin fungicides, sulfonanilide fungicides, sulfur, sultropen, TCMTB, tebuconazole, tecloftalam, tecnazene, tecoram, tetraconazole, thiabendazole, thiadifluor, thiazole fungicides, thicyofen, thifluzamide, thiocarbamate fungicides, thiochlorfenphim, thiomersal, thiophanate, thiophanate-methyl, thiophene fungicides, thioquinox, thiram, tiadinil, tioxymid, tivedo, tolclofos-methyl, tolnaftate, tolylfluanid, tolylmercury acetate, triadimefon, triadimenol, triamiphos, triarimol, triazbutil, triazine fungicides, triazole fungicides, triazoxide, tributyltin oxide, trichlamide, tricyclazole, tridemorph, trifloxystrobin, triflumizole, triforine, triticonazole, unclassified fungicides, undecylenic acid, uniconazole, urea fungicides, validamycin, valinamide fungicides, vinclozolin, zarilamid, zinc naphthenate, zineb, ziram, zoxamide and their mixtures.

***Herbicides.*** An herbicide is a pesticide used to kill unwanted plants. Selective herbicides kill specific targets while leaving the desired crop relatively unharmed. Some of these act by interfering with the growth of the weed and are often based on plant hormones. Herbicides used to clear waste ground are nonselective and kill all plant material with which they come into contact. Herbicides are widely used in agriculture and in landscape turf management. They are applied in total vegetation control (TVC) programs for maintenance of highways and railroads. Smaller quantities are used in forestry, pasture systems, and management of areas set aside as wildlife habitat. In the following, a number of suitable herbicides are compiled:
○ 2,4-D, a broadleaf herbicide in the phenoxy group used in turf and in no-till field crop production. Now mainly used in a blend with other herbicides that act as synergists, it is the most widely used herbicide in the world, third most commonly used in the United States. It is an example of synthetic auxin (plant hormone).
○ Atrazine, a triazine herbicide used in corn and sorghum for control of broadleaf weeds and grasses. It is still used because of its low cost and because it works as a synergist when used with other herbicides, it is a photosystem II inhibitor.
○ Clopyralid, a broadleaf herbicide in the pyridine group, used mainly in turf, rangeland, and for control of noxious thistles. Notorious for its ability to persist in compost. It is another example of synthetic auxin.
○ Dicamba, a persistent broadleaf herbicide active in the soil, used on turf and field corn. It is another example of synthetic auxin.
○ Glyphosate, a systemic nonselective (it kills any type of plant) herbicide used in no-till bumdown and for weed control in crops that are genetically modified to resist its effects. It is an example of a EPSPs inhibitor.
○ Imazapyr, a non-selective herbicide used for the control of a broad range of weeds including terrestrial annual and perennial grasses and broadleaved herbs, woody species, and riparian and emergent aquatic species.
○ Imazapic, a selective herbicide for both the pre- and post-emergent control of some annual and perennial grasses and some broadleaf weeds. Imazapic kills plants by inhibiting the production of branched chain amino acids (valine, leucine, and isoleucine), which are necessary for protein synthesis and cell growth.
○ Metoalachlor, a pre-emergent herbicide widely used for control of annual grasses in corn and sorghum; it has largely replaced atrazine for these uses.
○ Paraquat, a nonselective contact herbicide used for no-till burndown and in aerial destruction of marijuana and coca plantings. More acutely toxic to people than any other herbicide in widespread commercial use.
○ Picloram, a pyridine herbicide mainly used to control unwanted trees in pastures and edges of fields. It is another synthetic auxin.
○ Triclopyr.

***Insecticides.*** An insecticide is a pesticide used against insects in all developmental forms. They include ovicides and larvicides used against the eggs and larvae of insects. Insecticides are used in agriculture, medicine, industry and the household. In the following, suitable insecticides are mentioned:
○ Chlorinated insecticides such as, for example, Camphechlor, DDT, Hexachlorocyclohexane, gamma-Hexachlorocyclohexane, Methoxychlor, Pentachlorophenol, TDE, Aldrin, Chlordane, Chlordecone, Dieldrin, Endosulfan, Endrin, Heptachlor, Mirex and their mixtures;
○ Organophosphorus compounds such as, for example, Acephate, Azinphos-methyl, Bensulide, Chlorethoxyfos, Chlorpyrifos, Chlorpyriphos-methyl, Diazinon, Dichlorvos (DDVP), Dicrotophos, Dimethoate, Disulfoton, Ethoprop, Fenamiphos, Fenitrothion, Fenthion, Fosthiazate, Malathion, Methamidophos, Methidathion, Methyl-parathion, Mevinphos, Naled, Omethoate, Oxydemeton-methyl, Parathion, Phorate, Phosalone, Phosmet, Phostebupirim, Pirimiphos-methyl, Profenofos, Terbufos, Tetrachlorvinphos, Tribufos, Trichlorfon and their mixture;
○ Carbamates such as, for example, Aldicarb, Carbofuran, Carbaryl, Methomyl, 2-(1-Methylpropyl)phenyl methylcarbamate and their mixtures;
○ Pyrethroids such as, for example, Allethrin, Bifenthrin, Deltamethrin, Permethrin, Resmethrin, Sumithrin, Tetramethrin, Tralomethrin, Transfluthrin and their mixtures;
○ Plant toxin derived compounds such as, for example, Derris (rotenone), Pyrethrum, Neem (Azadirachtin), Nicotine, Caffeine and their mixtures.

***Rodenticides.*** Rodenticides are a category of pest control chemicals intended to kill rodents. Rodents are difficult to kill with poisons because their feeding habits reflect their place as scavengers. They would eat a small bit of something and wait, and if they do not get sick, they would continue eating. An effective rodenticide must be tasteless and odorless in lethal concentrations, and have a delayed effect. In the following, examples for suitable rodenticides are given:
○ Anticoagulants are defined as chronic (death occurs after 1 - 2 weeks post ingestion of the lethal dose, rarely sooner), single-dose (second generation) or multiple dose (first generation) cumulative rodenticides. Fatal internal bleeding is caused by lethal dose of anticoagulants such as brodifacoum, coumatetralyl or warfarin. These substances in effective doses are antivitamins K, blocking the enzymes K₁-2,3-epoxide-reductase (this enzyme is preferentially blocked by 4-hydroxycoumarin/4-hydroxythiacoumarin derivatives) and K₁-quinone-reductase (this enzyme is preferentially blocked by indandione derivatives), depriving the organism of its source of active vitamin K₁. This leads to a disruption of the vitamin K cycle, resulting in an inability of production of essential blood-clotting factors (mainly coagulation factors II (prothrombin), VII (proconvertin), IX (Christmas factor) and X (Stuart factor)). In addition to this specific metabolic disruption, toxic doses of 4-hydroxycoumarin/4-hydroxythiacoumarin and indandione anticoagulants are causing damage to tiny blood vessels (capillaries), increasing their permeability, causing diffuse internal bleedings (haemorrhagias). These effects are gradual; they develop in the course of days and are not accompanied by any nociceptive perceptions, such as pain or agony. In the final phase of intoxication the exhausted rodent collapses in hypovolemic circulatory shock or severe anemia and dies calmly. Rodenticidal anticoagulants are either first generation agents (4-hydroxycoumarin type: warfarin, coumatetralyl; indandione type: pindone, diphacinone, chlorophacinone), generally requiring higher concentrations (usually between 0.005 and 0.1%), consecutive intake over days in order to accumulate the lethal dose, poor active or inactive after single feeding and less toxic than second generation agents, which are derivatives of 4-hydroxycoumarin (difenacoum, brodifacoum, bromadiolone and flocoumafen) or 4-hydroxy-1-benzothiin-2-one (4-hydroxy-1-thiacoumarin, sometimes incorrectlly referred to as 4-hydroxy-1-thiocoumarin, for reason see heterocyclic compounds), namely difethialone. Second generation agents are far more toxic than first generation agents, they are generally applied in lower concentrations in baits (usually in the order of 0.001 - 0.005%), and are lethal after single ingestion of bait and are effective also against strains of rodents that have become resistant against first generation anticoagulants; thus the second generation anticoagulants are sometimes referred to as "superwarfarins". Sometimes, anticoagulant rodenticides are potentiated by an antibiotic, most commonly by sulfaquinoxaline. The aim of this association (e.g. warfarin 0.05% + sulfaquinoxaline 0.02%, or difenacoum 0.005% + sulfaquinoxaline 0.02% etc.) is that the antibiotic/bacteriostatic agent suppresses intestinal/gut symbiotic microflora that represents a source of vitamin K. Thus the symbiotic bacteria are killed or their metabolism is impaired and the production of vitamin K by them is diminuted, an effect which logically contributes to the action of anticoagulants. Antibiotic agents other than sulfaquinoxaline may be used, for example co-trimoxazole, tetracycline, neomycin or metronidazole. A further synergism used in rodenticidal baits is that of an association of an anticoagulant with a compound with vitamin D-activity, i.e. cholecalciferol or ergocalciferol (see below). A typical formula used is, e. g., warfarin 0.025 - 0.05% + cholecalciferol 0.01%. In some countries there are even fixed three-component rodenticides, i.e. anticoagulant + antibiotic + vitamin D, e. g. difenacoum 0.005% + sulfaquinoxaline 0.02% + cholecalciferol 0.01%. Associations of a second-generation anticoagulant with an antibiotic and/or vitamin D are considered to be effective even against the most resistant strains of rodents, though some second generation anticoagulants (namely brodifacoum and difethialone), in bait concentrations of 0.0025 - 0.005% are so toxic that no known resistant strain of rodents exists and even rodents resistant against any other derivatives are reliably exterminated by application of these most toxic anticoagulants.
   Vitamin K₁ has been suggested and successfully used as an antidote for pets or humans, which/who were either accidentally or intentionally (poison assaults on pets, suicidal attempts) exposed to anticoagulant poisons. In addition, since some of these poisons act by inhibiting liver functions and in progressed stages of poisoning, several blood-clotting factors as well as the whole volume of circulating blood lacks, a blood transfusion (optionally with the clotting factors present) can save a person's life who inadvertently takes them, which is an advantage over some older poisons.
○ Metal phosphides have been used as a means of killing rodents and are considered single-dose fast acting rodenticides (death occurs commonly within 1-3 days after single bait ingestion). A bait consisting of food and a phosphide (usually zinc phosphide) is left where the rodents can eat it. The acid in the digestive system of the rodent reacts with the phosphide to generate the toxic phosphine gas. This method of vermin control has possible use in places where rodents are resistant to some of the anticoagulants, particularly for control of house and field mice; zinc phosphide baits are also cheaper than most second-generation anticoagulants, so that sometimes, in cases of large infestation by rodents, their population is initially reduced by copious amounts of zinc phosphide bait applied, and the rest of the population that survived the initial fast-acting poison is then eradicated by prolonged feeding on anticoagulant bait. Inversely, the individual rodents that survived anticoagulant bait poisoning (rest population) can be eradicated by pre-baiting them with nontoxic bait for a week or two (this is important to overcome bait shyness, and to get rodents used to feeding in specific areas by offering specific food, especially when eradicating rats) and subsequently applying poisoned bait of the same sort as used for pre-baiting until all consumption of the bait ceases (usually within 2-4 days). These methods of alternating rodenticides with different modes of action provides a factual or an almost 100% eradication of the rodent population in the area if the acceptance/palatability of bait is good (i.e., rodents readily feed on it).
○ Phosphides are rather fast acting rat poisons, resulting in that the rats are dying usually in open areas instead of the affected buildings. Typical examples are aluminum phosphide (fumigant only), calcium phosphide (fumigant only), magnesium phosphide (fumigant only) and zinc phosphide (in baits). Zinc phosphide is typically added to rodent baits in amounts of around 0.75-2%. The baits have a strong, pungent garlic-like odor characteristic for phosphine liberated by hydrolysis. The odor attracts (or, at least, does not repulse) rodents, but has a repulsive effect on other mammals; birds, however (notably wild turkeys), are not sensitive to the smell and feed on the bait thus becoming collateral damage.
○ Hypercalcemia. Calciferols (vitamins D), cholecalciferol (vitamin D₃) and ergocalciferol (vitamin D₂) are used as rodenticides, which are toxic to rodents for the same reason that they are beneficial to mammals: they are affecting calcium and phosphate homeostasis in the body. Vitamins D are essential in minute quantities (few IUs per kilogram body weight daily, which is only a fraction of a milligram), and like most fat soluble vitamins they are toxic in larger doses as they readily result in the so-called hypervitaminosis, which is, simply said, poisoning by the vitamin. If the poisoning is severe enough (that is, if the dose of the toxicant is high enough), it eventually leads to death. In rodents consuming the rodenticidal bait it causes hypercalcemia by raising the calcium level, mainly by increasing calcium absorption from food, mobilising bone-matrix-fixed calcium into ionised form (mainly monohydrogencarbonate calcium cation, partially bound to plasma proteins, [CaHCO₃]⁺), which circulates dissolved in the blood plasma, and after ingestion of a lethal dose the free calcium levels are raised sufficiently so that blood vessels, kidneys, the stomach wall and lungs are mineralised/calcificated (formation of calcificates, crystals of calcium salts/complexes in the tissues thus damaging them), leading further to heart problems (myocard is sensitive to variations of free calcium levels that are affecting both myocardial contractibility and excitation propagation between atrias and ventriculas) and bleeding (due to capillary damage) and possibly kidney failure. It is considered to be single-dose, or cumulative (depending on concentration used; the common 0.075% bait concentration is lethal to most rodents after a single intake of larger portions of the bait), sub-chronic (death occurring usually within days to one week after ingestion of the bait). Applied concentrations are 0.075% cholecalciferol and 0.1% ergocalciferol when used alone. There is an important feature of calciferols toxicology which is that they are synergistic with anticoagulant toxicants. This means that mixtures of anticoagulants and calciferols in the same bait are more toxic than the sum of toxicities of the anticoagulant and the calciferol in the bait so that a massive hypercalcemic effect can be achieved by substantially lower calciferol content in the bait and vice-versa. More pronounced anticoagulant/hemorrhagic effects are observed if calciferol is present. This synergism is mostly used in baits low in calciferol because effective concentrations of calciferols are more expensive than effective concentrations of most anticoagulants. The historically very first application of a calciferol in rodenticidal bait was, in fact, the Sorex product Sorexa® D (with a different formula than today's Sorexa® D) back in the early 1970's, containing warfarin 0.025% + ergocalciferol 0.1%. Today, Sorexa® CD contains a 0.0025% difenacoum + 0.075% cholecalciferol combination. Numerous other brand products containing either calciferols 0.075 - 0.1% (e. g. Quintox®, containing 0.075% cholecalciferol) alone, or a combination of calciferol 0.01 - 0.075% with an anticoagulant are marketed.

***Miticides, moluscicides and nematicides.*** Miticides are pesticides that kill mites. Antibiotic miticides, carbamate miticides, formamidine miticides, mite growth regulators, organochlorine, permethrin and organophosphate miticides all belong to this category. Molluscicides are pesticides used to control mollusks, such as moths, slugs and snails. These substances include metaldehyde, methiocarb and aluminium sulfate. A nematicide is a type of chemical pesticide used to kill parasitic nematodes (a phylum of worm). A nematicide is obtained from a neem tree's seed cake; which is the residue of neem seeds after oil extraction. The neem tree is known by several names in the world but was first cultivated in India since ancient times.

***Antimicrobials.*** In the following examples, antimicrobials suitable for agrochemical compositions according to the present invention are given. Bactericidal disinfectants mostly used are those applying
○ active chlorine (i.e., hypochlorites, chloramines, dichloroisocyanurate and trichloroisocyanurate, wet chlorine, chlorine dioxide, etc.),
○ active oxygen (peroxides such as peracetic acid, potassium persulfate, sodium perborate, sodium percarbonate and urea perhydrate),
○ iodine (iodpovidone (povidone-iodine, Betadine), Lugol's solution, iodine tincture, iodinated nonionic surfactants),
○ concentrated alcohols (mainly ethanol, 1-propanol, called also n-propanol and 2-propanol, called isopropanol and mixtures thereof; further, 2-phenoxyethanol and 1- and 2-phenoxypropanols are used),
○ phenolic substances (such as phenol (also called "carbolic acid"), cresols (called "Lysole" in combination with liquid potassium soaps), halogenated (chlorinated, brominated) phenols, such as hexachlorophene, triclosan, trichlorophenol, tribromophenol, pentachlorophenol, Dibromol and salts thereof),
○ cationic surfactants such as some quaternary ammonium cations (such as benzalkonium chloride, cetyl trimethylammonium bromide or chloride, didecyldimethylammonium chloride, cetylpyridinium chloride, benzethonium chloride) and others, non-quarternary compounds such as chlorhexidine, glucoprotamine, octenidine dihydrochloride, etc.),
○ strong oxidizers such as ozone and permanganate solutions;
○ heavy metals and their salts such as colloidal silver, silver nitrate, mercury chloride, phenylmercury salts, copper sulfate, copper oxide-chloride etc. Heavy metals and their salts are the most toxic and environmentally hazardous bactericides and, therefore, their use is strongly suppressed or forbidden; further, also
○ properly concentrated strong acids (phosphoric, nitric, sulfuric, amidosulfuric, toluenesulfonic acids) and
○ alcalis (sodium, potassium, calcium hydroxides) between pH < 1 or > 13, particularly below elevated temperatures (above 60°C) kill bacteria.
   As antiseptics (i.e., germicide agents that can be used on human or animal body, skin, mucoses, wounds and the like), few of the above mentioned disinfectants can be used under proper conditions (mainly concentration, pH, temperature and toxicity toward man/animal). Among them, important are
○ Some properly diluted chlorine preparations (e. g. Daquin's solution, 0.5% sodium or potassium hypochlorite solution, pH-adjusted to pH 7 - 8, or 0.5 - 1% solution of sodium benzenesulfochloramide (chloramine B)), some
○ iodine preparations such as iodopovidone in various galenics (ointments, solutions, wound plasters), in the past also Lugol's solution,
○ peroxides as urea perhydrate solutions and pH-buffered 0.1 - 0.25% peracetic acid solutions,
○ alcohols with or without antiseptic additives, used mainly for skin antisepsis,
○ weak organic acids such as sorbic acid, benzoic acid, lactic acid and salicylic acid
○ some phenolic compounds such as hexachlorophene, triclosan and Dibromol, and
○ cation-active compounds such as 0.05 - 0.5% benzalkonium, 0.5 - 4% chlorhexidine, 0.1 - 2% octenidine solutions.

Bactericidal antibiotics kill bacteria; bacteriostatic antibiotics only slow down their growth or reproduction. Penicillin is a bactericide, as are cephalosporins. Aminoglycosidic antibiotics can act in both a bactericidic manner (by disrupting cell wall precursor leading to lysis) or bacteriostatic manner (by connecting to 30s ribosomal subunit and reducing translation fidelity leading to inaccurate protein synthesis). Other bactericidal antibiotics according to the present invention include the fluoroquinolones, nitrofurans, vancomycin, monobactams, co-trimoxazole, and metronidazole.

### Examples

### Example 1

### Saccharose+10 EO

An aqueous solution of 572 g (1.67 Mol) saccharose in 181 ml water and 4 g of an aqueous potassium hydroxide solution (50 % b.w.) were placed in a stirred autoclave. Once the reactor was three times evacuated and purged with nitrogen to remove all traces of oxygen, the mixture was heated to about 125 °C and within about 3.5 h 737 g (16,75 Mol) ethylene oxide was added, while the pressure raised to about 5.5 bar. Subsequently the mixture was left for another 30 min for post reaction, maintaining the temperature at about 130 °C., Finally, the reactor was cooled down to room temperature, vacuum was broken and the pH of the products adjusted to about 7 by adding a lactic acid solution. The liquid thus obtained consisted of about 88 % of Saccharose+10EO and about 12 % water; the PGE content has been less than 1 % b.w. Cf. 1 shows the MALDI spectra of the product.

### Example 2

### Herbicide compositions

Table 1 reflects a couple of herbicide compositions applicable according to instructions to use Monsanto's ROUND-UP, which are well known for those skilled in the art.

**Table 1**

| Herbicide compositions (all amounts in % b.w.) | | | | | | | | |
|---|---|---|---|---|---|---|---|---|
| **Components** | **1** | **2** | **3** | **4** | **5** | **6** | **7** | **8** |
| Glyphosate | 45 | 45 | 45 | 42 | 45 | 50 | - | - |
| Glufosinate | - | - | - | - | - | - | 45 | - |
| Paraquat | - | - | - | - | - | - | - | 45 |
| Saccharose+5EO | 15 | - | - | - | - | - | - | - |
| Saccharose+10EO | - | 15 | - | - | - | - | - | - |
| Saccharose+15EO | - | - | 15 | - | - | - | - | - |
| Maltose+10EO | - | - | - | 18 | 15 | 10 | - | - |
| Maltotriose+12EO | - | - | - | - | - | - | 15 | 15 |
| Water | add to 100 | | | | | | | |

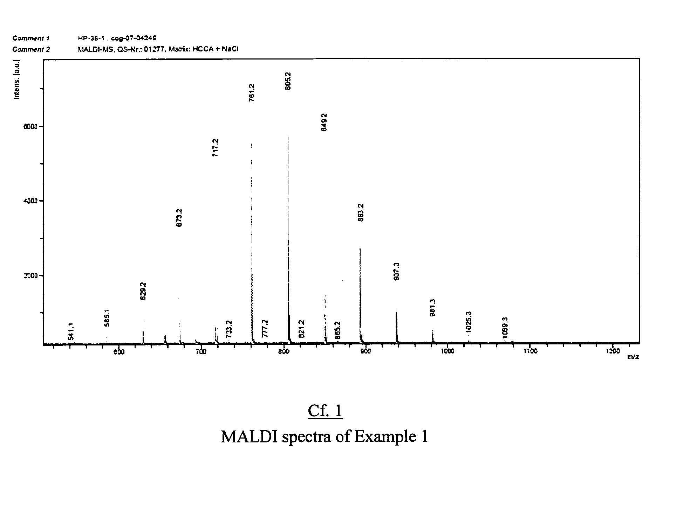

## Claims

1. Alkylene oxide adducts of oligosaccharides with decreased content of polyglycolethers, obtainable in than an aqueous solution of at least one water-soluble oligosaccharide is subjected to alkoxylation within a temperature range of 90 to 160 °C.

2. Process for making alkylene oxide adducts of oligosaccharides, **characterised in that** an aqueous solution of at least one water-soluble oligosaccharide is subjected to alkoxylation within a temperature range of 90 to 160 °C.

3. Process according to Claim 2, **characterised in that** water-soluble oligosaccharides are use which are selected from the group comprising saccharose, maltose and maltotriose.

4. Process according to Claim 2, **characterised in that** water-soluble oligosaccharides are used which are obtainable by chemical or enzymatic degradation of polysaccharides.

5. Process according to Claim 4, **characterised in that** said degradation products are obtained from celluloses, starches or waste material from sugar industry.

6. Process according to Claims 2 and/or 3, **characterised in that** alkoxylation is conducted by means of ethylene oxide, propylene oxide or their mixtures.

7. Process according to any of the preceding Claims 2 to 4, **characterised in that** alkoxylation is conducted with a mixture of ethylene oxide and propylene oxide, either blockwise or randomised.

8. Process according to any of the preceding Claims 2 to 5, **characterised in that** per each Mol oligosaccharide on average 1 to 50 Moles alkylene oxide are added.

9. Use of alkylene oxide adducts of oligosaccharides according to Claim 1 as additives in agriculture.

10. Use of alkylene oxide adducts as adjuvants, tank-mix adjuvants or formulation aids for making plant and crop protection compositions.

11. Agricultural compositions, comprising
(a) alkylene oxide adducts of oligosaccharides and
(b) biocides.

12. Agricultural compositions, comprising - calculated on the active matter -
(a) 1 to 50 % b.w. alkylene oxide adducts of oligosaccharides and
(c) 50 to 99 % b.w. biocides.
